# EUROPEAN PATENT SPECIFICATION

(11) **EP 0 934 241 B1**
(45) Date of publication and mention of the grant of the patent: **14.03.2001**
(21) Application number: 96933787.2
(22) Date of filing: 13.09.1996
(51) Int. Cl.: C07C 41/16, C07C 43/225

(54) **PRODUCTION OF 6-BROMO-2-METHOXYNAPHTHALENE AND DERIVATIVES**
HERSTELLUNG VON 6-BROM-2-METHOXYNAPHTHALIN UND DERIVATEN
PRODUCTION DE 6-BROMO-2-METHOXYNAPHTALENE ET DE SES DERIVES

(43) Date of publication of application: 11.08.1999
(73) Proprietor: ALBEMARLE CORPORATION, Baton Rouge, LA 70801-1765 (US)
(72) Inventor: SABAHI, Mahmood, Baton Rouge, LA 70810 (US)
(74) Representative: UEXKÜLL & STOLBERG
(86) International application number: US9614778
(87) International publication number: WO9811045

(56) References cited:
- GB-A- 1 561 025
- US-A- 5 225 603
- US-A- 5 536 870
- HELVETICA CHIMICA ACTA, vol. 68, no. 5, 14 August 1985, BASEL, CH, pages 1406-1426, XP002030770 U.H. LAUK, ET AL.: "Synthese und flüssigkristalline Eigenschaften 2,6-disubsituierter Naphthaline"

## Description

This invention relates to processes for preparing 6-bromo-2-methoxynaphthalene and derivatives thereof from 6-bromo-2-naphthol.

### Background

6-Bromo-2-methoxynaphthalene is a compound which has particular value as an intermediate in the preparation of non-steroidal antiinflammatory agents, such as 4-(6-methoxy-2-naphthyl)-2-butanone (commonly known as nabumetone) and 2-(6-methoxy-2-naphthyl)-propionic acid (commonly known as naproxen).

It is known that 6-bromo-2-methoxynaphthalene can be prepared by reacting 6-bromo-2-naphthol with a 260% stoichiometric excess of dimethyl sulfate in the presence of aqueous sodium hydroxide. Excellent yields are obtained by this process, but it has the disadvantage of generating an aqueous waste stream containing hazardous sodium methyl sulfate.

Lewis et al., "Methylation of Phenol by Dimethyl Sulfate," *Industrial and Engineering Chemistry,* Vol. 22, pp. 34-36 (1930), suggest that production of sodium methyl sulfate could be minimized and the yield of the desired product improved by avoiding the use of excessive amounts of water in the reaction between a phenol and dimethyl sulfate. However, their experimental results show that they were unable to get product yields as high as 90% in less than five hours - regardless of the water/ phenol mol ratio they used.

GB-A-1 561 025 discloses a process for the production of 6-bromo-2-methoxy naphthalene by reacting 6-bromo-2-naphthol with 1.4 molar proportions of dimethyl sulhate in the presence of 1.6 molar proportions of sodium hydroxide and 82 molar proportions of water.

It would be desirable to find a way of rapidly reacting 6-bromo-2-naphthol with dimethyl sulfate to prepare 6-bromo-2-methoxynaphthalene in high yields without the co-formation of sodium methyl sulfate.

### Summary of Invention

It has now been found that 6-bromo-2-naphthol can be rapidly reacted with dimethyl sulfate to prepare 6-bromo-2-methoxynaphthalene in high yields without the co-formation of sodium methyl sulfate when the reaction is conducted in the presence of controlled amounts of sodium hydroxide and water and in an organic solvent. Thus, the invention resides in a process which comprises reacting 6-bromo-2-naphthol with dimethyl sulfate in the presence of substantially stoichiometric amounts of sodium hydroxide and water and in an organic solvent and subsequently, if desired, converting the 6-bromo-2-alkoxynaphthlene product to a pharmaceutically-active derivative, such as nabumetone or naproxen. Embodiments of this invention include:
(1) a process for preparing 6-bromo-2-methoxynaphthaleneby reacting 6-bromo-2-naphthol with dimethyl sulfate in the presence of substantially stoichiometric amounts of sodium hydroxide and water and in an organic solvent and
(2) processes for preparing nabumetone or naproxen from a 6-bromo-2-methoxynaphthalene obtained by reacting 6-bromo-2-naphthol with dimethyl sulfate in the presence of substantially stoichiometric amounts of sodium hydroxide and water and in an organic solvent.

### Detailed Description

The amount of dimethyl sulfate reacted with 6-bromo-2-naphthol in the practice of the invention does not appear to be critical to attaining some improvements by controlling the concentrations of sodium hydroxide and water in the reaction mixture. However, it is desirable to use a stoichiometric excess of the sulfate in order to obtain high yields while avoiding the use of as large an excess as has previously been required in known dimethyl sulfate/6-bromo-2-naphthol reactions to attain high yields. Thus, it is ordinarily preferred for the dimethyl sulfate/6-bromo-2-naphthol mol ratio in the reaction mixture to be in the range of 0.6-0.8/1, preferably 0.7/1.

As already mentioned, the sodium hydroxide and water ingredients of the reaction mixture are utilized in "substantially stoichiometric amounts," i.e., amounts that are either the precisely stoichiometric amounts or amounts that do not greatly differ from the precisely stoichiometric amounts - ordinarily amounts that do not differ from the exactly stoichiometric amounts by more than 15%. Thus, in the practice of the invention, the reaction between 6-bromo-2-naphthol and dimethyl sulfate may be conducted in the presence of 1-1.15 mols of sodium hydroxide and 1-1.15 mols of water per mol of 6-bromo-2-naphthol.

Because of the small amount of water permitted in the reaction, the sodium hydroxide is incorporated in solid form - ordinarily as pellets or flakes.

Organic solvents utilizable in the reaction between the 6-bromo-2-naphthol and dimethyl sulfate include a variety of solvents, such as hydrocarbons, halogenated hydrocarbons, alcohols, and ethers. However, hydrocarbons (especially aromatic hydrocarbons such as benzene, toluene, and xylenes) and halogenated hydrocarbons (e.g., brominated and chlorinated aromatic hydrocarbons and halogenated aliphatic hydrocarbons, such as ethylene dichloride) are usually preferred.

The novel dimethyl sulfate/6-bromo-2-naphthol reaction may be conducted at any temperature which permits the reaction to occur but is preferably conducted at a temperature in the range of 80-115°C to complete the reaction in 1-2 hours.

After completion of the sulfate/6-bromo-2-naphthol reaction, the reaction mixture containing the 6-bromo-2-methoxynaphthalene product can be worked up in any conventional manner to remove impurities and any solvent that the practitioner wishes to separate from the product.

Derivatives of the 6-bromo-2-methoxynaphthalene product of the foregoing process can be prepared by any processes suitable for preparing those derivatives from 6-bromo-2-methoxynaphthalene. Such processes, of course, are already well known. For example:
(1) As described in U.S. Patent 5,225,603 (Aslam et al.), the 6-bromo-2-methoxynaphthalene may be (a) reacted with N,N-dimethylformamide to form 6-methoxy-2-naphthaldehyde, which is then condensed with acetone to prepare a 4-(6-methoxy-2-naphthyl)-3-buten-2-one intermediate that is then catalytically hydrogenated to nabumetone or (b) reacted with methyl vinyl ketone to form the 4-(6-methoxy-2-naphthyl)-3-buten-2-one intermediate that is then catalytically hydrogenated to nabumetone.
(2) As disclosed in U.S. Patent 5,536,870 (Wu), the 6-bromo-2-methoxy-naphthalene may be reacted with ethylene to form 2-methoxy-6-vinylnapthalene, which is then carbonylated to racemic 2-(6-methoxy-2-naphthyl)propionic acid, which can be subsequently resolved to isolate the desired naproxen enantiomer.

Both U.S 5,225,603 and U.S. 5,536,870 are incorporated herein by reference as if fully set forth.

The invention is particularly advantageous in that it (a) permits dimethyl sulfate to be used as the methylating agent in preparing 6-bromo-2-methoxynaphthalene from 6-bromo-2-naphthol without generating toxic sodium methyl sulfate, (b) lowers the cost of the 6-bromo-2-naphthol/dimethyl sulfate reaction both by reducing the amount of dimethyl sulfate required to obtain high yields and by eliminating the need to dispose of a waste stream containing toxic sodium methyl sulfate, and (c) allows the reaction to be completed in as little as 1-2 hours.

The following examples are given to illustrate the invention and are not intended as a limitation thereof.

### EXAMPLE 1

Add 3.6g (0.2 mol) of water, 17.6g (0.14 mol) of dimethyl sulfate, and 9.0g (0.22 mol) of sodium hydroxide to a solution of 44.6g (0.2 mol) of 6-bromo-2-naphthol in 140 mL of ethylene dichloride. Heat the mixture to 100°C, maintain that temperature for two hours, cool to room temperature, add 50 mL of water, and neutralize the aqueous phase with dilute acid. Analysis of the organic phase shows 95% 6-bromo-2-methoxynaphthalene, 1% unreacted 6-bromo-2-naphthol, 1% 2-bromo-6-chloroethoxynaphthalene, and 1% methoxynaphthalene.

### EXAMPLE 2

Charge a reactor with 22.3g (0.1 mol) of 6-bromo-2-naphthol, 80 mL of toluene, 4.5g (0.11 mol) of sodium hydroxide pellets, 1.8g (0.1 mol) of water, and 8.8g (0.07 mol) of dimethyl sulfate. After refluxing the reaction mixture for one hour and cooling to room temperature, add 50 mL of water and separate the aqueous layer. Analysis of the organic layer shows 95% 6-bromo-2-methoxynaphthalene, 2% 6-bromo-2-naphthol, and 2% 5-methyl-6-bromo-2-methoxynaphthalene.

## Claims

1. A process which comprises reacting 6-bromo-2-naphthol with an dimethyl sulfate to prepare 6-bromo-2-methoxynaphthalene and optionally then converting the 6-bromo-2-methoxynaphthalene to a derivative, characterized in that the reaction between the 6-bromo-2-naphthol and dimethyl sulfate is conducted in the presence of substantially stoichiometric amounts of sodium hydroxide and water and in an organic solvent.

2. The process of claim 1 which is a process for preparing 6-bromo-2-methoxy-naphthalene.

3. The process of claim 1 which is a process for preparing 4-(6-methoxy-2-naphthyl)-2-butanone by reacting 6-bromo-2-naphthol with dimethyl sulfate in the presence of substantially stoichiometric amounts of sodium hydroxide and water and in an organic solvent to form 6-bromo-2-methoxynaphthalene, and then converting the 6-bromo-2-methoxynaphthalene thus obtained to 4-(6-methoxy-2-naphthyl)-2-butanone.

4. The process of claim 1 which is a process for preparing 2-(6-methoxy-2-naphthyl)propionic acid by reacting 6-bromo-2-naphthol with dimethyl sulfate in the presence of substantially stoichiometric amounts of sodium hydroxide and water and in an organic solvent to form 6-bromo-2-methoxynaphthalene, and then converting the 6-bromo-2-methoxynaphthalene thus obtained to 2-(6-methoxy-2-naphthyl)propionic acid.

5. A process according to claim 1 wherein the derivative is 4-(6-methoxy-2-naphthyl)-2-butanone obtained by reacting 6-bromo-2-naphthol with a dimethylsulphate.

6. A process according to claim 1 wherein 6-bromo-2-methoxynaphthalene is further converted to the derivative 2-(6-methoxy-2-naphthyl)propionic acid.

7. The process of any of claims 1-6 wherein the organic solvent used in the reaction between 6-bromo-2-naphthol and dimethyl sulfate is an aromatic hydrocarbon.

8. The process of any of claims 1-6 wherein the organic solvent used in the reaction between 6-bromo-2-naphthol and dimethyl sulfate is a halogenated hydrocarbon.

9. The process of any of the preceding claims wherein the 6-bromo-2-methoxynaphthalene is prepared by reacting one molar proportion of 6-bromo-2-naphthol with 0.6-0.8 molar proportion of dimethyl sulfate in the presence of 0.85-1.15 molar proportions of sodium hydroxide and 0.85-1.15 molar proportions of water and in an organic solvent.

## Patentansprüche

1. Verfahren, bei dem 6-Brom-2-naphthol mit Dimethylsulfat umgesetzt wird, um 6-Brom-2-methoxynaphthalin herzustellen, und dann gegebenenfalls das 6-Brom-2-methoxynaphthalin in ein Derivat umgewandelt wird, dadurch gekennzeichnet, dass die Reaktion zwischen dem 6-Brom-2-naphthol und dem Dimethylsulfat in Gegenwart von im Wesentlichen stöchiometrischen Mengen von Natriumhydroxid und Wasser und in einem organischen Lösungsmittel durchgeführt wird.

2. Verfahren nach Anspruch 1, das ein Verfahren zur Herstellung von 6-Brom-2-methoxynaphthalin ist.

3. Verfahren nach Anspruch 1, das ein Verfahren zur Herstellung von 4-(6-Methoxy-2-naphthyl)-2-butanon durch Umsetzung von 6-Brom-2-naphthol mit Dimethylsulfat in Gegenwart von im Wesentlichen stöchiometrischen Mengen von Natriumhydroxid und Wasser und in einem organischen Lösungsmittel zur Bildung von 6-Brom-2-methoxynaphthalin ist, bei dem dann das so erhaltene 6-Brom-2-methoxynaphthalin in 4-(6-Methoxy-2-naphthyl)-2-butanon umgewandelt wird.

4. Verfahren nach Anspruch 1, das ein Verfahren zur Herstellung von 2-(6-Methoxy-2-naphthyl)propionsäure durch Umsetzung von 6-Brom-2-naphthol mit Dimethylsulfat in Gegenwart von im Wesentlichen stöchiometrischen Mengen von Natriumhydroxid und Wasser und in einem organischen Lösungsmittel zur Bildung von 6-Brom-2-methoxynaphthalin ist, bei dem dann das so erhaltene 6-Brom-2-methoxynaphthalin in 2-(6-Methoxy-2-naphthyl)propionsäure umgewandelt wird.

5. Verfahren nach Anspruch 1, bei dem das Derivat 4-(6-Methoxy-2-naphthyl)-2-butanon ist, das durch Umsetzung von 6-Brom-2-naphthol mit Dimethylsulfat erhalten worden ist.

6. Verfahren nach Anspruch 1, bei dem 6-Brom-2-methoxynaphthalin weiter in das Derivat 2-(6-Methoxy-2-naphthyl)propionsäure umgewandelt wird.

7. Verfahren nach einem der Ansprüche 1 bis 6, bei dem das organische Lösungsmittel, das bei der Reaktion zwischen 6-Brom-2-naphthol und Dimethylsulfat verwendet wird, ein aromatischer Kohlenwasserstoff ist.

8. Verfahren nach einem der Ansprüche 1 bis 6, bei dem das organische Lösungsmittel, das bei der Reaktion zwischen 6-Brom-2-naphthol und Dimethylsulfat verwendet wird, ein halogenierter Kohlenwasserstoff ist.

9. Verfahren nach einem der vorhergehenden Ansprüche, bei dem das 6-Brom-2-methoxynaphthalin durch Umsetzung von einem molaren Anteil von 6-Brom-2-naphthol mit 0,6 bis 0,8 molaren Anteilen von Dimethylsulfat in Gegenwart von 0,85 bis 1,15 molaren Anteilen Natriumhydroxid und 0,85 bis 1,15 molaren Anteilen Wasser und in einem organischen Lösungsmittel hergestellt wird.

## Revendications

1. Procédé qui comprend la réaction du 6-bromo-2-naphtol avec le sulfate de diméthyle pour préparer le 6-bromo-2-méthoxynaphtalène, puis, facultativement, la conversion du 6-bromo-2-méthoxynaphtalène en un dérivé, caractérisé en ce que la réaction entre le 6-bromo-2-naphtol et le sulfate de diméthyle est conduite en présence de quantités sensiblement stoechiométriques d'hydroxyde de sodium et d'eau et dans un solvant organique.

2. Procédé de la revendication 1, qui est un procédé destiné à préparer le 6-bromo-2-méthoxynaphtalène.

3. Procédé de la revendication 1, qui est un procédé destiné à préparer la 4-(6-méthoxy-2-naphtyl)-2-butanone par réaction du 6-bromo-2-naphtol avec le sulfate de diméthyle en présence de quantités sensiblement stoechiométriques d'hydroxyde de sodium et d'eau et dans un solvant organique pour former le 6-bromo-2-méthoxynaphtalène, puis conversion du 6-bromo-2-méthoxynaphtalène ainsi obtenu en 4-(5-méthoxy-2-naphtyl)-2-butanone.

4. Procédé de la revendication 1, qui est un procédé destiné à préparer l'acide 2-(6-méthoxy-2-naphtyl)propionique par réaction du 6-bromo-2-naphtol avec le sulfate de diméthyle en présence de quantités sensiblement stoechiométriques d'hydroxyde de sodium et d'eau et dans un solvant organique pour former le 6-bromo-2-méthoxynaphtalène, puis conversion du 6-bromo-2-méthoxynaphtalène ainsi obtenu en acide 2-(6-méthoxy-2-naphtyl)propionique.

5. Procédé selon la revendication 1, dans lequel le dérivé est la 4-(6-méthoxy-2-naphtyl)-2-butanone obtenue par réaction du 6-bromo-2-naphtol avec le sulfate de diméthyle.

6. Procédé selon la revendication 1, dans lequel le 6-bromo-2-méthoxynaphtalène est de plus converti en le dérivé acide 2-(6-méthoxy-2-naphtyl)propionique.

7. Procédé de l'une quelconque des revendications 1 à 6, dans lequel le solvant organique utilisé dans la réaction entre le 6-bromo-2-naphtol et le sulfate de diméthyle est un hydrocarbure aromatique.

8. Procédé de l'une quelconque des revendications 1 à 6, dans lequel le solvant organique utilisé dans la réaction entre le 6-bromo-2-naphtol et le sulfate de diméthyle est un hydrocarbure halogéné.

9. Procédé de l'une quelconque des revendications précédentes, dans lequel le 6-bromo-2-méthoxynaphtalène est préparé en faisant réagir 1 proportion molaire de 6-bromo-2-naphtol avec 0,6 à 0,8 proportion molaire de sulfate de diméthyle en présence de 0,85 à 1,15 proportion molaire d'hydroxyde de sodium et 0,85 à 1,15 proportion molaire d'eau et dans un solvant organique.
